(19) Europäisches Patentamt
European Patent Office
Office européen des brevets

(11) Publication number: **0 394 039 A1**

# (12) EUROPEAN PATENT APPLICATION

(21) Application number: **90304209.1**

(22) Date of filing: **19.04.90**

(51) Int. Cl.[5]: **C07D 211/58, C07D 409/06, C07D 401/06**

(30) Priority: **20.04.89 US 340976**

(43) Date of publication of application: **24.10.90 Bulletin 90/43**

(84) Designated Contracting States: **AT BE CH DE DK ES FR GB GR IT LI LU NL SE**

(71) Applicant: **BOC, INC.**
**575 Mountain Avenue**
**Murray Hill New Jersey 07974(US)**

(72) Inventor: **Lin, Bor-Sheng**
**86 Briarwood Drive**
**Berkeley Heights, New Jersey 07922(US)**
Inventor: **Spencer, H. Kenneth**
**35 Hilltop Terrace**
**Chatham, New Jersey 07928(US)**
Inventor: **Scheblein, Joseph W.**
**45 Adams Court**
**Flemington, New Jersey 08822(US)**

(74) Representative: **Wickham, Michael et al**
**c/o Patent and Trademark Department The BOC Group plc Chertsey Road**
**Windlesham Surrey GU20 6HJ(GB)**

## (54) A method for preparing 4-Alkoxyalkyl-4-aminophenylpiperidines and derivatives thereof.

(57) The present invention is directed to a method for preparing an 4-alkoxyalkyl-4-aminophenylpiperidine compound which comprises the steps of (a) reacting an N-substituted-4-piperidone compound with an aniline compound to form a Schiff base compound, (b) reacting the Schiff base compound with an anionic reagent having an anion stabilizing group to form an amine compound, and (c) reducing the amine compound in step (b) with a reducing agent to displace the anion stabilizing group.

The anionic reagent in step (b) above has the general formula:

X-CYM-Z

wherein X is a member selected from the group consisting of anion stabilizing groups, Y is a member selected from the group consisting of hydrogen and lower-alkyl, Z is a member selected from the group consisting of lower-alkoxy and phenylmethoxy, M is a metal atom selected from the group consisting of alkali and alkaline earth metals, and C is a carbon atom.

EP 0 394 039 A1

## A METHOD FOR PREPARING 4-ALKOXYALKYL-4-AMINOPHENYLPIPERIDINES AND DERIVATIVES THEREOF

The present invention relates to a method for preparing 4-alkoxyalkyl-4aminophenylpiperidines and N-phenyl-N-(4-alkoxy-alkylpiperidin-4-yl)amides and derivatives thereof, useful as intravenous analgesics or anaesthetics.

A number of patents disclose certain N-phenyl-N-(4-alkoxyalkylpiperidin-4-yl)amides having therapeutic activity. For example, United States Patent No. 3,998,834 discloses certain N-phenyl-N-[(N-heterocyclic)-piperidin-4-yl]amide compounds, useful as analgesics, having a 4-lower-alkoxy methyl substituent on the piperidine ring. United States Patent No. 4,584,303 also discloses certain 4-alkoxyalkyl-substituted N-phenyl-N-[(N-heterocyclic)piperidin-4-yl] -amide compounds, useful as analgesics, having a 4-lower-alkoxy methyl substituent on the piperidine ring.

The 4-lower-alkoxy methyl piperidine ring substituent in these prior art compounds is introduced by reacting an appropriately N-substituted-4-piperidone compound with an appropriately substituted aniline compound and an alkali metal cyanide. The resulting nitrile derivative is then hydrolyzed to the corresponding amide, which in turn is hydrolyzed to the corresponding acid, which is then esterified, reduced with a hydride reagent to the corresponding alcohol, and subsequently O-alkylated.

V. H. Rawal et al. disclose the use of the alpha-lithium derivative of methoxymethyl phenyl sulphide, [-(methoxymethyl)-thio]benzene, as a synthetic reagent useful in adding to certain compounds containing a carbonyl group, a halide group, or an epoxide group, as well as to certain compounds containing a nitrile group, an amide group, or an acid chloride group, to form the corresponding acyl derivative compound, Synthetic Communications, 14, 1129-1139 (1984).

The present invention is directed to a method for preparing an 4-alkoxyalkyl-4-aminophenylpiperidine compound which comprises the steps of (a) reacting an N-substituted-4-piperidone compound with an aniline (aminophenyl) compound to form a Schiff base compound, (b) reacting the Schiff base compound with an anionic reagent having an anion stabilizing group to form an amine compound, and (c) reducing the amine compound in step (b) with a reducing agent to displace the anion stabilizing group, wherein the anionic reagent in step (b) above has the general formula:

X-CYM-Z

wherein X is a member selected from the group consisting of anion stabilizing groups, Y is a member selected from the group consisting of hydrogen and lower-alkyl, Z is a member selected from the group consisting of lower-alkoxy and phenylmethoxy, M is a metal atom selected from the group consisting of alkali and alkaline earth metals, and C is a carbon atom.

In a preferred embodiment, the present invention is directed to a method for preparing 4-alkoxyalkyl-4-aminophenylpiperidine compounds and N-aminophenyl-N-(4-alkoxyalkylpiperidin-4-yl)amide derivatives having the general formula:

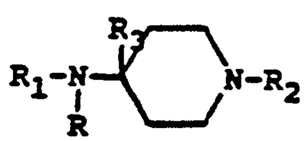

(I)

wherein R is a member selected from the group consisting of phenyl and substituted phenyl; $R_1$ is a member selected from the group consisting of hydrogen, lower-alkyl carbonyl, lower-alkenyl carbonyl, lower-alkoxy lower-alkyl carbonyl and cycloalkyl carbonyl; $R_2$ is a member selected from the group consisting of hydrogen, lower-alkyl, cycloalkylmethyl, phenyl lower-alkyl, and heterocyclic ring system lower-alkyl; and $R_3$ is a member selected from the group consisting of lower-alkoxy lower-alkyl and phenylmethoxy lower-alkyl.

Several convenient routes for the preparation of the compounds which may be prepared by the method of the present invention begin with the known piperidone starting materials shown below:

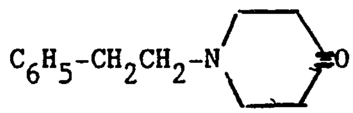

(1)

or

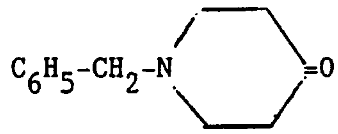

(2)

Compound (1), N-(2-phenylethyl)-4-piperidone, can be prepared according to the procedure published by A. H. Becket, A.F. Casey and G. Kirk, J. Med Pharm. Chem., Vol. 1, p. 37 (1959). Compound (2), N-phenylmethyl-4-piperidone, can be prepared in an analogous manner by the procedure described by C.R. Ganellin and R.G. Spickch, J. Med. Chem., Vol. 8; p. 619 (1965) or P.M. Carabateas and L. Grumbach, J. Med. Pharm. Chem., Vol. 5, p. 913 (1962).

In one example of the method of the present invention, an N-substituted-4- piperidone compound, such as N-(2-phenyl-ethyl)-4-piperidone (1), is reacted with aniline, or a substituted aniline, to form a Schiff base compound (3).

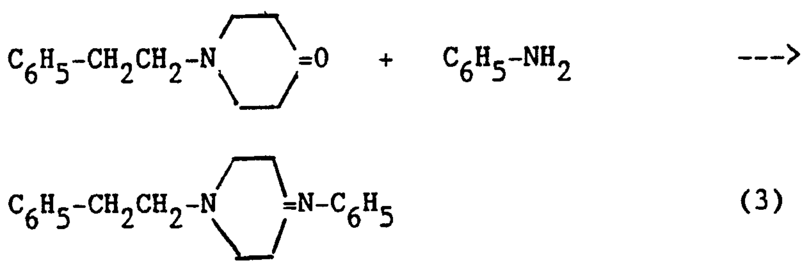

(3)

The Schiff base compound (3) is then reacted with an anionic reagent (4) having the general formula:

X-CYM-Z (4)

wherein X is a member selected from the group consisting of anion stabilizing groups, Y is a member selected from the group consisting of hydrogen and lower-alkyl, Z is a member selected from the group consisting of lower-alkoxy and phenylmethoxy, M is a metal atom selected from the group consisting of alkali and alkaline earth metals, and C is a carbon atom.

The Schiff base compound (3) and anionic reagent (4), such as the alpha-lithium derivative of methoxymethyl phenyl sulphide, react to form compound (5) 4-aminophenyl-4-(1-thiophenyl-1-methoxymethyl)-N-(2-phenylethyl) piperidine.

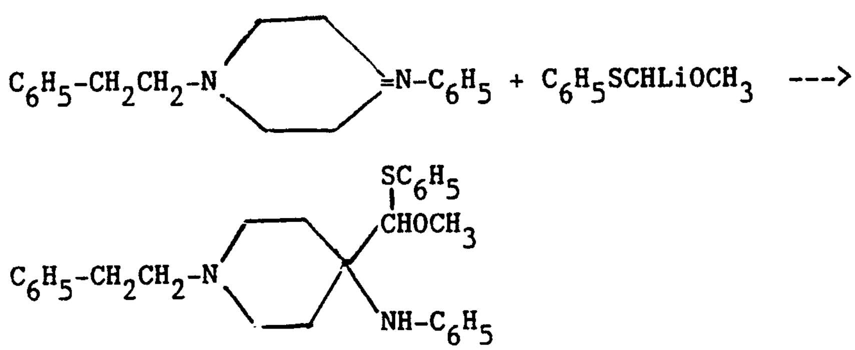

(5)

Amine compound (5) is then reduced with a suitable reducing agent, for example, n-tributyltin hydride, to displace the thiophenyl anion stabilizing group and yield 4-aminophenyl-4-methoxymethyl-N-(2-

phenylethyl)piperidine (6).

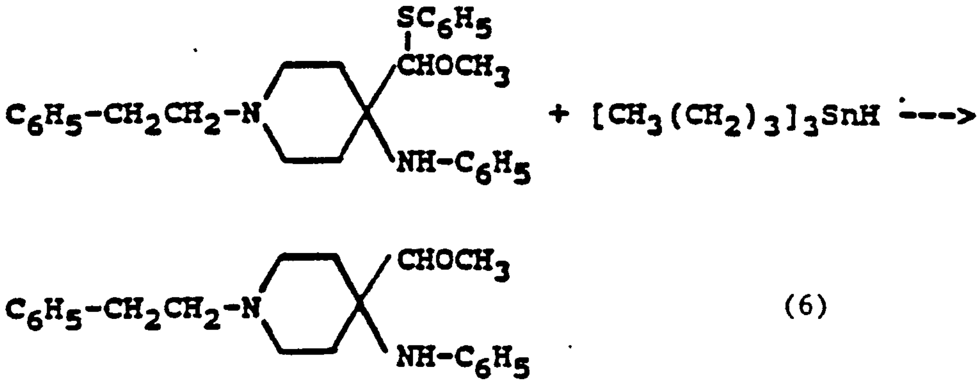


(6)

Compound (6) can be reacted with an appropriate acid halide (e.g., $R_1COCl$) or an anhydride (e.g., $(R_1CO)_2O$) to introduce the desired $R_1$ group on the nitrogen atom and thereby obtain compound (I), according to the reaction scheme shown below:

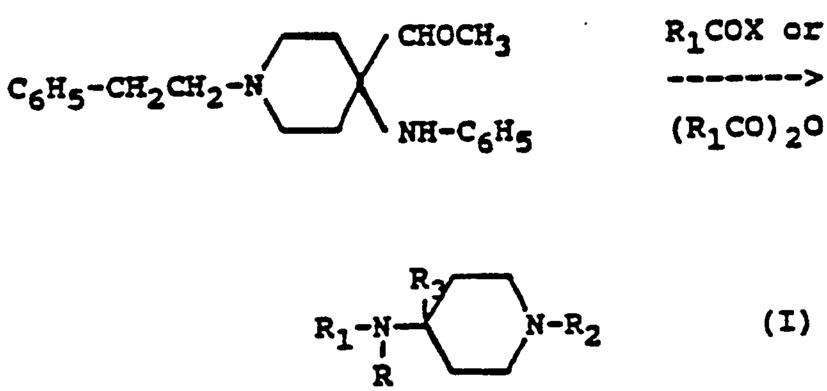


(I)

wherein $R_1$, $R_2$ and $R_3$ are defined as set forth above.

The anionic reagent of the present invention has the general formula:

X-CYM-Z (4)

therein X, Y, Z, M and C are defined as set forth below:

In Formula 4, X is a member selected from the group consisting of anion stabilizing groups. The anion stabilizing groups of the present invention are electronegative groups which can stabilize a negative charge on the adjacent carbon atom C such that carbon atom C can add to Schiff base compounds of Formula (3). Suitable anion stabilizing groups may be selected from the group consisting of thiophenyl ($C_6H_5S$-), sulfinylphenyl ($C_6H_5SO$-), sulfonylphenyl ($C_6H_5SO_2$-), other thioaryl, sulfinylaryl, and sul-fonylaryl groups, and the like, and tertiary alkylthio groups. In a preferred embodiment, the anion stabilizing group is thiophenyl or sulfonylphenyl.

Y in Formula 4 above is a member selected from the group consisting of hydrogen and lower-alkyl. In a preferred embodiment, the Y group is selected from the group consisting of hydrogen, methyl, and ethyl. In a more preferred embodiment, the Y group is hydrogen or methyl.

Z in Formula 4 above is selected from the group consisting of lower-alkoxy and phenylmethoxy. In a preferred embodiment, the Z group is selected from the group consisting of methoxy, ethoxy, n-propanoxy, and phenylmethoxy. In a more preferred embodiment, the Z group is methoxy or ethoxy.

M in Formula 4 above is a metal atom selected from the group consisting of alkali and alkaline earth metals. In a preferred embodiment, M is selected from the group consisting of lithium, sodium, and potassium. In a more preferred embodiment, M is lithium or sodium.

In a preferred embodiment, the anionic reagent of formula (4) is selected from the group consisting of

the alpha-lithium derivatives of methoxymethyl phenyl sulphide, $C_6H_5SCHLiOCH_3$, and methoxymethyl phenyl sulfoxide, $C_6H_5SO_2CHLiOCH_3$ especially the former.

The Schiff base compound (3) may be selected from a wide variety of compounds to prepare compounds of type (I). Suitable compounds of type (I) are set forth below.

Reaction conditions suitable for carrying out the addition of the anionic reagent (4) to the Schiff base compound (3) are disclosed in, for example, V. H. Rawal et al., Synthetic Communications, 14, 1129-1139 (1984), which disclosure is incorporated herein by reference.

Suitable reducing agents in the present invention include Raney nickel, metal hydrides, and sodium amalgam. Suitable metal hydrides include n-tributyltin hydride and triphenyltin hydride. In a preferred embodiment, the reducing agent is a member selected from the group consisting of n-tributyltin hydride and triphenyltin hydride.

When the desired $R_2$ substituent group is not phenylethyl, one procedure for preparing compounds of the present invention with different $R_2$ groups is to begin with compounds of type (2) according to the following scheme:

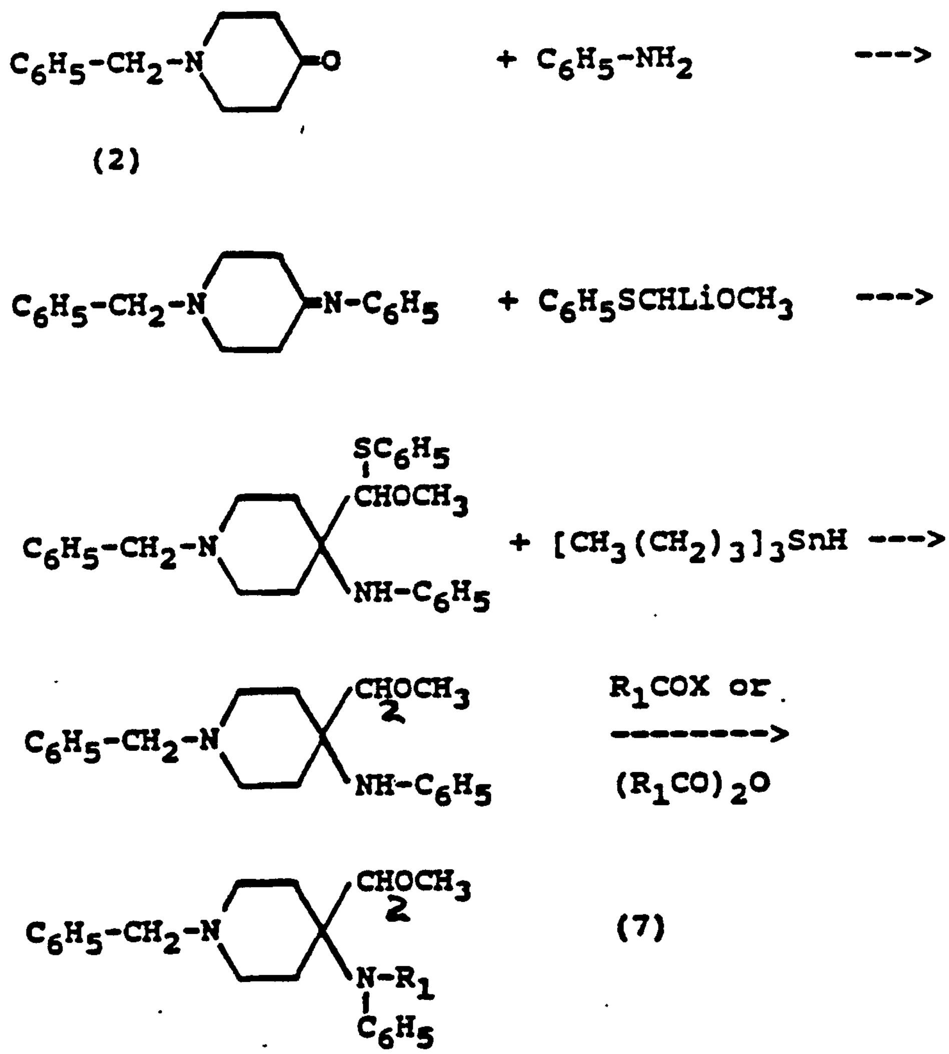

(2)
(7)

The phenylmethyl group in compound (7) can be removed by hydrogenolysis, or by reaction with 1-chloroethyl chloro-formate followed by hydrolysis with methanol. The preparation of secondary amines of type (8) has been described by P. G. H. Van Daele et al., Arzneim-Forsch. Drug Res., 26, p. 1521, (1976) and R.A. Olofson et al., J. Org. Chem., 49, pp. 2081-2082 (1984). The phenylmethyl group can be replaced with a desired $R_2$ substituent group by reacting compound (8) with an appropriately reactive molecule $R_2$-X, wherein X is halogen, such as chlorine, bromine, or iodine, or its reactive equivalent, such as toluene

sulfonate, phenyl sulfonate, methyl sulfonate, and the like, to obtain compound (I) according to the reaction scheme illustrated below:

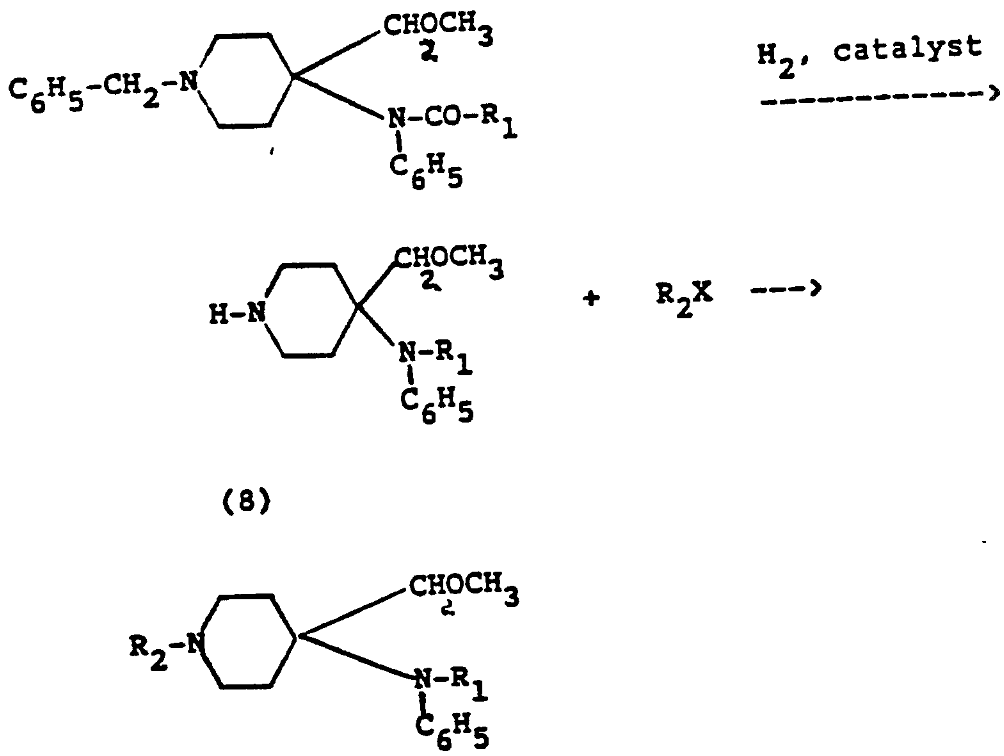


(8)

(I)

The reaction of $R_2$-X with a piperidinyl intermediate of type (8) can be conducted in an inert organic solvent such as, for example, an aromatic hydrocarbon, a ketone such as 4-methyl-2-pentanone and the like, an ether such as 1,4-dioxane, diethylether, tetrahydofuran, 1,2-dimethoxyethane and the like, or N,N-dimethylformamide. The addition of an appropriate base, such as an alkali metal carbonate, may be utilized to neutralize the acid generated during the reaction. The addition of an iodide salt, such as an alkali metal iodide, may be appropriate. The temperature of the reaction mixture may be raised to increase the rate of reaction when appropriate.

The procedures for preparing compounds of type (I), which include a variety of therapeutic agents, notably sufentanil and alfentanil, which are used in humans for inducing anesthesia or analgesia, are disclosed in detail in United States patent no. 4,584,303, issued to Huang, et al., and United States patent no. 3,998,834, issued to Janssen et al., which are incorporated herein by reference.

The compounds which may be prepared by the method of the present invention possess very desirable analgesic activities. In particular, these compounds produce central nervous system depressant effects including analgesia, hypnosis, sedation, increased pain threshold, and barbiturate and/or general anesthetic potentiation. Many of the compounds provide highly potent analgesia with immediate onset and a short duration of activity. These properties are highly desirable in circumstances where acute pain must be eliminated over a short period of time, such as in anesthesiology. The preferred compounds provide reduced rigidity at high doses, superior motor coordination recovery, or less respiratory depressive and/or cardio-vascular depressive activity when compared to fentanyl, (N-phenyl-N-[1-(2-phenylethyl)-4-piperidinyl]propanamide).

As set out above, the present invention is directed to a method for preparing 4-alkoxyalkyl-4-aminophenyl-piperidine compounds. In a preferred embodiment, the invention is directed to a method for preparing certain opioid 4-alkoxy-alkyl-4-aminophenylpiperidine compounds and N-aminophenyl-N-(4-alkoxyalkylpiperidin-4-yl)amide derivatives having the general formula:

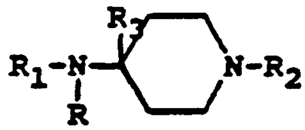

(I)

including optically active isomeric forms, wherein R is a member selected from the group consisting of phenyl and substituted phenyl; $R_1$ is a member selected from the group consisting of hydrogen, lower-alkyl carbonyl, lower-alkenyl carbonyl, lower-alkoxy lower-alkyl carbonyl and cycloalkyl carbonyl; $R_2$ is a member selected from the group consisting of hydrogen, lower-alkyl, cycloalkylmethyl, phenyl lower-alkyl, and heterocyclic ring system lower-alkyl; and $R_3$ is a member selected from the group consisting of lower-alkoxy lower-alkyl and phenylmethoxy lower-alkyl.

Group R (which is contributed by the said aniline compound) in formula I above is an aryl group selected from the group consisting of phenyl and substituted phenyl, wherein the substituents on the phenyl group are members independently selected from the group consisting of halogen, lower-alkyl, lower-alkoxy, and combinations thereof. The preferred substituents are selected from the group consisting of fluoro and methoxy. The preferred position for attachment of a substituent to the phenyl ring is at the 2 (ortho) position. In a preferred embodiment, the R group is a member selected from the group consisting of phenyl, 2-fluorophenyl and 2-methoxyphenyl.

Group $R_1$ in formula I above is a member selected from the group consisting of hydrogen, lower-alkyl carbonyl, lower-alkenyl carbonyl, lower-alkoxy carbonyl and cycloalkyl carbonyl, each alkyl group having from 2 to 6 carbon atoms. In a preferred embodiment, the $R_1$ group is a member selected from the group consisting of ethyl carbonyl, ethenyl carbonyl, methoxy methyl carbonyl and alpha-methoxy ethyl carbonyl.

Group $R_2$ in formula I above is a member selected from the group consisting of hydrogen, lower-alkyl, cyclo-alkylmethyl, phenyl lower-alkyl, and heterocyclic ring system lower-alkyl. The phenyl lower-alkyl group may be unsubstituted or substituted, wherein the substituents on the phenyl ring are selected from the group consisting of halogen, lower alkoxy, lower alkyl and combinations thereof. The heterocyclic lower-alkyl ring systems may be selected from the group consisting of monocyclic heterocyclic lower-alkyl ring systems having 5 to 6 ring member atoms and fused bicyclic and tricyclic heterocyclic lower-alkyl ring systems having 5 to 6 ring member atoms in each ring of the polycyclic ring system. The heteroatom is a member selected from the group consisting of nitrogen, sulfur and oxygen.

In a preferred embodiment, the heterocyclic lower-alkyl ring system is a member selected from the group consisting of pyrrolyl lower-alkyl, pyrazolyl lower-alkyl, imidazolyl lower-alkyl, imidazolinyl lower-alkyl, imidazolyl lower-thioalkyl, triazolyl lower-alkyl, triazolyl lower-thioalkyl, tetrazolyl lower-alkyl, tetrazolyl lower-thioalkyl, thienyl lower-alkyl, thienyl lower-oxyalkyl, thienyl lower-hydroxy- alkyl, furanyl lower-hydroxyalkyl, thiazolyl lower-alkyl, oxazolyl lower-alkyl, thiadiazolyl lower-alkyl, oxadiazolyl lower-alkyl, pyridin-1-yl lower-alkyl, pyridin-3-yl lower-alkyl, pyridin-4-yl lower-alkyl, piperidinyl lower-alkyl, pyrimidinyl lower-alkyl, pyridazinyl lower-alkyl, triazinyl lower-alkyl, indolyl lower-alkyl, isoindolyl lower-alkyl, benzimidazolyl lower-alkyl, benzopyrazolyl lower-alkyl, benzoxazolyl lower-alkyl, benzopyranyl lower-alkyl, benzodioxanyl lower-alkyl, benzothiazinyl lower-alkyl, quinazolinyl lower-alkyl, purinyl lower-alkyl, phthalimidyl lower-alkyl, naphthalenecarboxamidyl lower-alkyl, and naphthalene-sulfamidyl lower-alkyl.

The heterocyclic ring may be unsubstituted or substituted, wherein the substituent group is a member independently selected from the group consisting of halogen, oxygen, hydroxyl, nitro, amino, carbonyl, lower-alkoxy carbonyl, lower-alkyl, lower-cycloalkyl, lower-alkoxy, lower-thioalkyl, halogenated lower-alkyl, aryl, halogenated aryl, heterocycles, and combinations thereof. In a preferred embodiment, the substituent group is a member selected from the group consisting of fluoro, chloro, iodo, oxygen, nitro, amino, carbonyl, ethoxy carbonyl, methyl, ethyl, isopropyl, methoxy, thiomethyl, trifluoromethyl, phenyl, morpholinyl and combinations thereof.

The lower-alkyl group is a member selected from the group consisting of branched or unbranched hydrocarbon groups containing from 1 to 7 carbon atoms (typically 1 to 6 carbon atoms). The lower-alkyl group may be substituted or unsubstituted, with substituent members independently selected from the group consisting of oxygen, hydroxyl, sulfur, and combinations thereof. In a preferred embodiment, the lower-alkyl group is a member selected from the group consisting of methyl, ethyl, 2-hydroxyethyl, 2-oxoethyl, and 2-thioethyl.

In a more preferred embodiment, the heterocyclic lower-alkyl ring system is a member selected from the group consisting of thiazolyl lower alkyl, which can be substituted in the 4-position with a lower-alkyl group, 4,5-dihydro-5-oxo-1H-tetrazol-1-yl lower alkyl, which can be substituted in the 4-position with a lower-alkyl group, and thienyl lower-alkyl.

Group $R_3$ in formula I above is a member selected from the group consisting of lower-alkoxy lower-alkyl and phenylmethoxy lower-alkyl. In a preferred embodiment, the $R_3$ group is a member selected from the group consisting of methoxy methyl, ethoxy methyl, methoxy ethyl and ethoxy ethyl. In a more preferred embodiment, the $R_3$ group is methoxy methyl.

In a most preferred embodiment, the invention is directed to a method for preparing certain opioid N-aminophenyl-N-(4-alkoxyalkylpiperidin-4-yl)amide derivatives having the general formula (I) wherein R is unsubstituted phenyl, $R_1$ is ethyl carbonyl, $R_2$ is a member selected from the group consisting of 2-(2-thienyl)ethyl and 2-(4-ethyl-4,5-dihydro-5-oxo-1H- tetrazol-1-yl)ethyl, and $R_3$ is methoxymethyl.

The term "lower-alkyl," as used herein, means branched or unbranched hydrocarbon groups containing from 1 to 7 carbon atoms (typically 1 to 6 carbon atoms), and preferably from 1 to 4 carbon atoms. The term "lower-alkoxy," as used herein, means branched or unbranched hydrocarboxy groups containing from 1 to 7 carbon atoms (typicaly 1 to 6 carbon atoms), and preferably from 1 to 4 carbon atoms. The term "lower-thioalkoxy," as used herein, means branched or unbranched hydrothiocarboxy groups containing from 1 to 7 carbon atoms (typically 1 to 6 carbon atoms), and preferably from 1 to 4 carbon atoms. The term "lower-cycloalkyl," as used herein, means cyclic alkyl groups containing from 3 to 6 carbon atoms. Preferred heterocyclic groups include from 6 to 12 carbon atoms and can include the substituents discussed above in connection with heterocyclic groups. The term halogen, as used herein, refers to the chemically related elements consisting of fluorine, chlorine, bromine and iodine, preferably fluorine.

The compounds of the present invention which have at least one asymmetric carbon atom can exist in optically active isomeric forms. For example, in compounds in which $R_2$ is a 2-phenyl-1-propyl or 1-phenyl-2-propyl group, etc., the carbon adjacent to the piperidinyl nitrogen is an asymmetric carbon atom and such compounds can therefore exist in optical active isomeric (enantiomeric) forms. Such isomeric forms can be isolated from the racemic mixtures by techniques known to those skilled in the art.

The present invention is further illustrated by the following examples which are presented for purposes of demonstrating, but not limiting, the preparation of the compounds of this invention.

## EXAMPLE 1

### 4-Aminophenyl-4-(1-thiophenyl-1-methoxy-methyl)-N-phenylmethyl piperidine

This Example illustrates a method for preparing a Schiff base compound and the subsequent reaction of the Schiff base compound with an anionic reagent to prepare an intermediate compound according to the present invention.

A solution of N-phenylmethyl-4-piperidone (3.78g, 20 mmole), aniline (1.87g, 20 mmole) and p-toluenesulfonic acid (100mg) in benzene (50ml) was heated to reflux in a two-neck flask fitted with a Dean-Stark water trap overnight. The resulting reddish brown reaction solution was then concentrated under vacuum and the Schiff base residue was dissolved in anhydrous tetrahydrofuran (50ml).

A solution of tert-butyllithium (24 mmole) in pentane was added dropwise to a solution of methoxymethyl phenyl sulphide (3.70g, 24 mmole) in anhydrous tetrahydrofuran (100ml) at -78° C. After addition, the resulting yellowish reaction solution was stirred for 1 hour at -78° C. The above Schiff base solution was added dropwise to the stirred lithium solution at -78° C. The resulting reaction solution was stirred at -78° C for 15 minutes and then at room temperature for 20 minutes. The reaction solution was then quenched by adding $H_2O$ (100ml). The resulting mixture was extracted with ethyl acetate (2 x 100ml) and the combined organic layers were dried over sodium sulfate and concen- trated under vacuum. The residue was chromatographed (silica gel; eluted with methylene chloride, then ethyl acetate/hexane, 1:3) to yield the product, 4-aminophenyl-4-(1-thiophenyl-1-methoxymethyl)- N-phenylmethyl piperidine, (5.35g, 12.8 mmole), in 64% yield as an amber oil.

## EXAMPLE 2

### 4-Aminophenyl-4-methoxymethyl-N-phenylmethyl piperidine

This Example illustrates a method for displacing the thiophenyl anion stabilizing group in the intermediate compound of Example 1 to prepare a compound according to the present invention.

Raney nickel (38g, wet, W-2, washed with ethanol, then ethyl acetate) was added to a solution of 4-aminophenyl-4-(1-thiophenyl-1-methoxymethyl)-N-phenyl-methyl piperidine (3.10g, 7.4 mmole) in ethyl acetate (100ml). The resulting mixture was stirred at room temperature for 1 hour. The reaction mixture was then filtered through a sintered glass funnel and the filtrate was concentrated under vacuum. The resulting residue was chromatographed (silica gel; ethyl acetate) to yield the product, 4-aminophenyl-4-methoxymethyl-N-phenylmethyl piperidine, (1.27g, 4.1 mmole) in 55% yield as a colorless oil.

## EXAMPLE 3

### 4-Aminophenyl-4-methoxymethyl-N-phenylmethyl piperidine

This Example illustrates another method for displacing the thiophenyl anion stabilizing group in the intermediate compound of Example 1 to prepare a compound according to the present invention.

A 5 liter, 3-neck round bottom flask affixed with a thermometer, a magnetic stirrer, and a condenser connected to a dry nitrogen gas inlet tube was charged with 89.7g (0.214 moles) of 4-aminophenyl-4-(thiophenyl-methoxy-methyl-N-phenylmethyl piperidine, 3.0g (18 mmoles) of azobisisobutyronitrile, 1.2 liters of toluene, and 307.1g (1.023 moles) of n-tributyltin hydride. The reaction mixture was slowly heated to reflux over a period of 1 hour and then maintained at this temperature for 22 hours. The reaction mixture was quenched by the addition of a solution of 50ml of concentrated hydrochloric acid and 50ml of methanol at a temperature from about 0° C. to about 5° C. The mixture was made alkaline by the addition of 20% sodium hydroxide solution (250g). The emulsified aqueous layer was extracted with toluene (3 x 150ml). The combined organic layers were washed with water (2 x 150ml), then dried over sodium sulfate, and concentrated under vacuum. The resulting residue was purified by column chromatography (silica gel; ethyl acetate/hexane, 1:9 to 1:1) to yield 56.5g (81.8% yield) of the product, 4-aminophenyl-4-methoxymethyl-N-phenylmethyl piperidine.

## Claims

1. A method for preparing an 4-alkoxyalkyl-4-aminophenylpiperidine compound comprising the steps of:
(a) reacting an N-substituted-4-piperidone compound with an aniline compound to form a Schiff base compound;
(b) reacting the Schiff base compound with an anionic reagent having an anion stabilizing group to form an amine compound; and
(c) reducing the amine compound in step (b) with a reducing agent to displace the anion stabilizing group;
wherein the anionic reagent in step (b) has the general formula:
X-CYM-Z
wherein X is a member selected from the group consisting of anion stabilizing groups; Y is a member selected from the group consisting of hydrogen and lower-alkyl; Z is a member selected from the group consisting of lower-alkoxy and phenylmethoxy; M is a metal atom selected from the group consisting of alkali and alkaline earth metals; and C is a carbon atom.

2. A method according to Claim 1, wherein X is a member selected from the group consisting of thiophenyl, sulfinylphenyl, and sulfonylphenyl.

3. A method according to Claim 1 or Claim 2, wherein Y is a member of the group consisting of hydrogen, methyl, and ethyl.

4. A method according to any one of the preceding claims, wherein Z is a member of the group consisting of methoxy, ethoxy, n-propanoxy, and phenylmethoxy.

5. A method according to any one of the preceding claims, wherein M is a member of the group consisting of lithium, sodium, and potassium.

6. A method according to any one of the preceding claims, wherein the anionic reagent is selected from the group consisting of the alpha-lithium derivatives of methoxymethyl phenyl sulphide and methoxymethyl phenyl sulfoxide.

7. A method according to any one of the preceding Claims, wherein the reducing agent is a member selected from the group consisting of Raney nickel, metal hydrides, and sodium amalgam.

8. A method according to Claim 7, wherein the reducing agent is a member selected from the group

consisting of n-tributyltin hydride and triphenyltin hydride.

9. A method of Claim 1, wherein the N-substituted- 4-piperidone compound is a member selected from the group consisting of N-phenylmethyl-4-piperidone and N-(2-phenyl- ethyl)-4-piperidone).

10. A method according to any one of the preceding claims, wherein the 4-alkoxyalkyl-4-aminophenylpiperidine compound has the general formula:

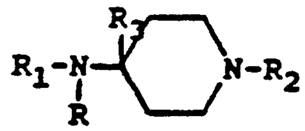

wherein R is a member of the group consisting of phenyl and substituted phenyls; $R_1$ is a member of the group consisting of hydrogen, lower-alkyl carbonyl, lower-alkenyl carbonyl, lower-alkoxy lower-alkyl carbonyl and cycloalkyl carbonyl; $R_2$ is a member of the group consisting of hydrogen, lower-alkyl, cycloalkylmethyl, phenyl lower/alkyl, and heterocyclic ring system lower-alkyl; and $R_3$ is a member of the group consisting of lower-alkoxy lower-alkyl and phenylmethoxy lower-alkyl.

11. A method according to Claim 10, wherein R is a member of the group consisting of phenyl, 2-fluorophenyl and 2-methoxyphenyl; $R_1$ is a member of the group consisting of ethyl carbonyl, ethenyl carbonyl, methoxy methyl carbonyl and methoxy ethyl carbonyl; $R_2$ is a member of the group consisting of hydrogen, lower-alkyl, cycloalkylmethyl, phenyl lower/alkyl, pyrrolyl lower-alkyl, pyrazolyl lower-alkyl, imidazolyl lower-alkyl, imidazolinyl lower-alkyl, imidazolyl lower-thioalkyl, triazolyl lower-alkyl, triazolyl lower-thioalkyl, tetrazolyl lower-alkyl, tetrazolyl lower-thioalkyl, thienyl lower-alkyl, thienyl lower-oxyalkyl, thienyl lower-hydroxyalkyl, furanyl lower-hydroxyalkyl, thiazolyl lower-alkyl, oxazolyl lower-alkyl, thiadiazolyl lower-alkyl, oxadiazolyl lower-alkyl, pyridin-1-yl lower-alkyl, pyridin-3-yl lower-alkyl, pyridin-4-yl lower-alkyl, piperidinyl lower-alkyl, pyrimidinyl lower-alkyl, pyridazinyl lower-alkyl, triazinyl lower-alkyl, indolyl lower-alkyl, isoindolyl lower-alkyl, benzimidazolyl lower-alkyl, benzopyrazolyl lower-alkyl, benzoxazolyl lower-alkyl, benzopyranyl lower-alkyl, benzodioxanyl lower-alkyl, benzothiazinyl lower-alkyl, quinazolinyl lower-alkyl, purinyl lower-alkyl, phthalimidyl lower-alkyl, naphthalenecarboxamidyl lower-alkyl, and naphthalene-sulfamidyl lower-alkyl; and $R_3$ is a member of the group consisting of methoxy methyl, ethoxy methyl, methoxy ethyl and ethoxy ethyl.

12. A method according to Claim 11, wherein R is phenyl; $R_1$ is ethyl carbonyl; $R_2$ is a member of the group consisting of 2-(2-thienyl)ethyl and 2-(4-ethyl-4,5-dihydro-5-oxo-1H-tetrazol-1-yl)ethyl; and $R_3$ is methoxymethyl.

13. A method according to Claim 10, wherein in said substituted phenyls the or each substituent is independently selected from halogens, lower alkyl groups and lower alkoxy groups and combinations thereof.

European Patent Office

# EUROPEAN SEARCH REPORT

Application number

EP 90304209.1

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| D,A | US - A - 4 584 303 (HUANG) * Claim 1; column 5, line 33 - column 6, line 60; column 9, line 58 - column 11, line 65 * -- | 1,7,9, 10 | C 07 D 211/58<br>C 07 D 409/06<br>C 07 D 401/06 |
| A | US - A - 3 164 600 (JANSSEN) * Claim 1; column 2, lines 4 -34 * -- | 1,7,9 | |
| A | CHEMICAL ABSTRACTS, vol. 104, no. 15, April 14, 1986 Columbus, Ohio, USA S. HACKETT et al. "(Methoxy (phenylthio)methyl)lithium and (methoxy(phenylthio)(trimethylsilyl)methyl)lithium" page 637, column 2, abstract -no. 129 121k & J.Org.Chem., 51(6), 879-85 (1986) ---- | 1,2-6 | TECHNICAL FIELDS SEARCHED (Int Cl 5)<br>C 07 D 211/00<br>C 07 D 409/00<br>C 07 D 401/00 |

The present search report has been drawn up for all claims

| Place of search | Date of completion of the search | Examiner |
|---|---|---|
| VIENNA | 23-07-1990 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document